# EUROPEAN PATENT SPECIFICATION

(11) **EP 0 238 905 B1**
(45) Date of publication and mention of the grant of the patent: **25.08.1993**
(21) Application number: 87103079.7
(22) Date of filing: 04.03.1987
(51) Int. Cl.: C07D 239/42, C07D 413/12, A61K 31/505, A61K 31/535, C07C 265/02, C07C 275/04

(54) **4-(2-pyrimidinyl)-1-piperazinyl heterocyclic carbonyl derivatives**
4-(2-Pyrimidyl)-1-piperazinyl-substituierte heterozyklische Carbonylderivate
Dérivés de carbonyle hétérocycliques substitués par un groupe 4-(2-pyrimidyl)-1-pipérazinyle

(30) Priority: 05.03.1986 US 836277
(43) Date of publication of application: 30.09.1987
(73) Proprietor: MERRELL DOW PHARMACEUTICALS INC., Cincinnati Ohio 45215-6300 (US)
(72) Inventor: Hibert, Marcel, F-67000 Strasbourg (FR); Gittos, Maurice W., F-67115 Plobsheim (FR)
(74) Representative: Sgarbi, Renato

(56) References cited:
- EP-A- 0 000 220
- EP-A- 0 129 128
- DE-A- 2 920 739
- US-A- 3 398 151
- US-A- 3 717 634

## Description

### FIELD OF THE INVENTION

This invention relates to certain 4-(2-pyrimidinyl)-1-piperazinyl heterocyclic carbonyl derivatives and their use as anxiolytic agents.

### BACKGROUND OF THE INVENTION

Anxiety has been defined as an apprehension or concern regarding some future event. Most, if not all, people occasionally suffer some symptoms of anxiety in response to appropriate stimuli. In some individuals, these feelings of anxiety or panic in response to the everyday pressures of life can be overwhelming, rendering the individual an unproductive member of society. Whereas individual group counseling represents the preferred primary mode of therapy, the use of chemotherapeutic agents has proven to be a useful adjunct in the treatment of anxiety, thereby enabling a seriously afflicted individual to regain productive status while undergoing concurrent psychotherapy.

Compounds of the class of benzodiazepines are currently the therapeutic agents of choice in the treatment of anxiety. In particular, chlordiazepoxide, diazepam and oxazepam are commonly used. This class of compounds has a great potential for misuse, particularly among the class of patients undergoing therapy. Moreover, the benzodiazepines generally possess undesired sedative effects and process detracting interactions with other drugs, including for example, alcohol.

Applicants have now discovered a class of novel 4-(2-pyrimidinyl)-1-piperazinyl heterocyclic carbonyl derivatives, which are useful as antianxiety agents, and which are generally free from the undesirable effects of the benzodiazepines. The compounds disclosed herein, when practiced in accordance with the teachings of this invention help to alleviate such symptoms as excessive fear, worry, restlessness, tension, stress, neurotic depression and are useful in the relief of some personality disorders.

EP-A 220 describes a class of hexahydropyrimidines that are reported to be serotonine antagonist and possess anti-aggregating, anti-hystaminic and psychotropic activities.

US-A 3398151 describes 8-(4-phenyl-1-piperazinylalkylene)-8-azaspiro[4,5]decane-7,9-diones as possessing psychotropic activity in animal tests.

US-A 3717634 describes N-(heterocyclic)-piperazinylalkyl derivatives of azaspiroalkanediones that are reported to be active on some animals tests that are considered predictive of tranquilizing and anti-emetic properties.

DE-A 2920739 describes 8-[4-[4-(2-pyrimidinyl)-1-piperazinyl]-butyl]-8-azaspiro[4,5]decan-7,9-diones as possessing anxiolytic activity.

### SUMMARY OF THE INVENTION

This invention is directed to a class of 4-(2-pyrimidinyl)-1-piperazinyl heterocyclic carbonyl derivatives having the formula 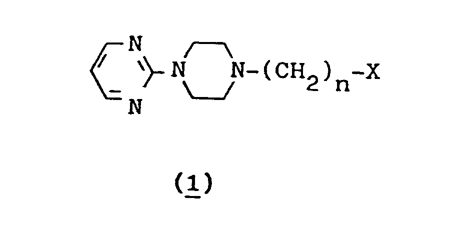
wherein n is an integer from 2 to 5; X is the radical 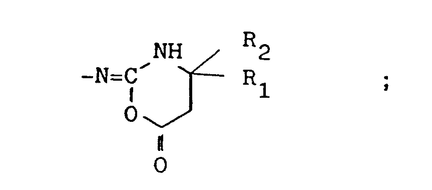
R₁ and R₂ are each methyl or when taken together form a tetramethylene or pentamethylene ring; and the pharmaceutically acceptable acid addition salts thereof.

This invention also relates to a process for the preparation of said compounds, pharmaceutical compositions thereof, and to the use of these compounds as anxiolytic agents.

### DETAILED DESCRIPTION OF THE INVENTION

As used herein the term "pharmaceutically acceptable acid addition salts" is intended to apply to any non-toxic organic or inorganic acid addition salts of the base compounds represented by Formula I. Illustrative inorganic acids which form suitable salts include hydrochloric, hydrobromic, sulphuric and phosphoric acid and acid metal salts such as sodium monohydrogen orthophosphate and potassium hydrogen sulfate. Organic acids which form suitable salts include the mono, di and tricarboxylic acids. Illustrative examples of such acids include: acetic, glycolic, lactic, pyruvic, malonic, succinic, glutaric, fumaric, malic, tartaric, citric, ascorbic, maleic, hydroxymaleic, benzoic, p-hydroxybenzoic, phenylacetic, cinnamic, salicylic, 2-phenoxybenzoic acids, and sulfonic acids, such as methanesulfonic acid or 2-hydroxy-ethanesulfonic acid. Either the mono or the di-acid salts can be formed, and such salts can exist in either a hydrated or a substantially anhydrous form. In general, the acid addition salts of these compounds are crystalline materials which are soluble in water and in various hydrophilic organic solvents. In comparison to their free base forms, such salts generally demonstrate higher melting points and an increase in chemical stability.

As can be seen in formula (1) above, the compounds of this invention consist essentially of two terminal heterocyclic moieties separated by an alkylene chain having from 2 to 5 carbon atoms. More particularly, one such heterocyclic moiety consists of the 4-(2-pyrimidinyl)-1-piperazinyl group, whereas the other heterocyclic moiety represents a tetrahydro-6H-1,3-oxazine-6-one. Still more particularly the compounds of this invention can be illustrated as follows: 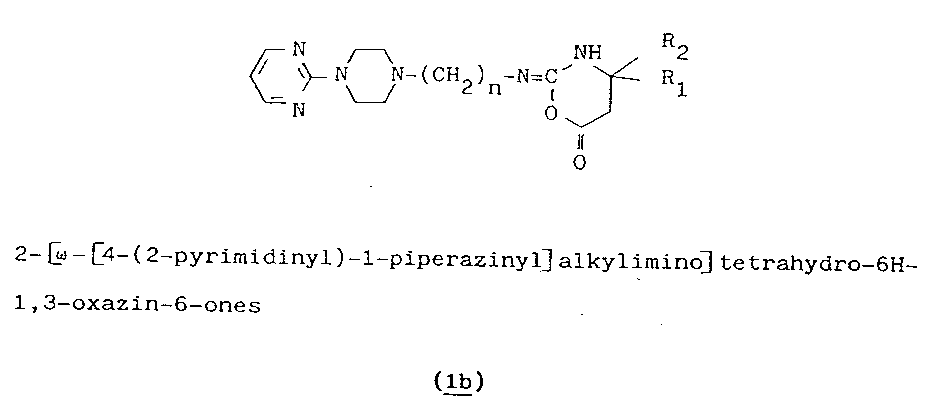
Those alkylene groups in which the symbol n is from 2-4 carbon atoms represent the preferred alkylene chain lengths. The symbols R₁ and R₂ are either methyl or when taken together can form a tetramethylene or pentamethylene ring, i.e., either a cyclopentane or a cyclohexane ring.

The compounds of formula (1b) are prepared via a procedure that prepares also the tetrahydropyrimidine-2,4-diones of formula (1a) 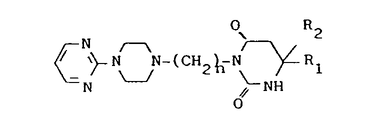
wherein R₁, R₂ and n have the same meanings defined above, that are not claimed in this application.

Thus, an alkyl ester of 3-[(N-alkyl)ureido]propionic acid (2) is condensed with the compound 1-(2-pyrimidinyl)piperazine (3) to form an alkyl ester of 3-[N-[ω-[4-(2-pyrimidinyl)-1-piperazinyl]alkyl]ureido]propionic acid (4). These alkyl esters (4) are generally not isolated, but are permitted to spontaneously cyclize to form the desired 1-[ω-[4-(2-pyrimidinyl)-1-piperazinyl]alkyl]-tetrahydro-pyrimidine- 2,4-diones (1a) and 2-[ω-[4-(2-pyrimidinyl)-1-piperazinyl]-alkylimino]tetrahydro-6H-1,3-oxazine-6-ones (1b). This reaction sequence can be illustrated as follows, wherein the symbols n, R₁ and R₂ are as previously described, R is a lower alkyl group having 1 to 4 carbon atoms and Y represents an appropriate leaving group. 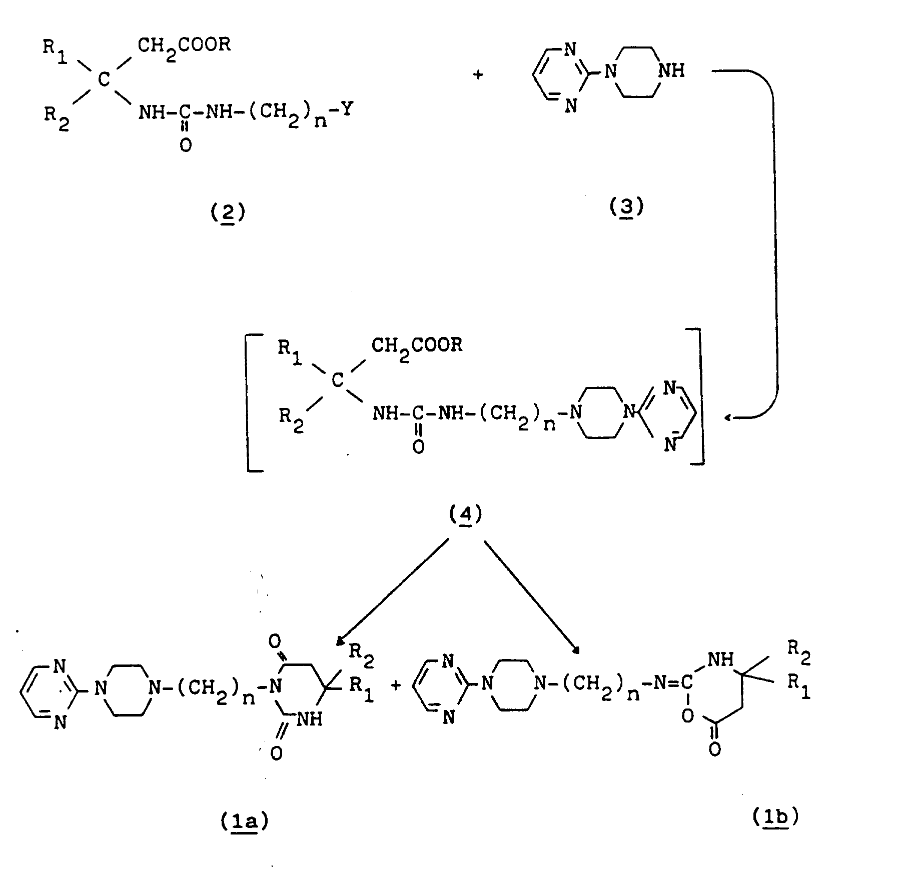
The term "lower alkyl", as indicated by the symbol R, represents an alkyl group having from 1 to 4 carbon atoms. Illustrative lower alkyl groups include methyl, ethyl, propyl, isopropyl, butyl and sec butyl. The term "leaving group", as indicated by the symbol Y, represents chlorine, bromine, iodine or the tosylate and mesylate groups.

The nucleophilic condensation is preferably conducted using equimolar amounts of 1-(2-pyrimidinyl)piperazine (3) with the alkyl ester of 3-[(N-alkyl)ureido]propionic acid (2) for a period of from about 1 hour to 24 hours depending upon the particular reactants employed. The reaction temperature can range from about 25°C to 140°C. Preferably the reaction is conducted at a temperature ranging from 60°C to 125°C.

The use of solvents is preferred. Suitable solvents include any non-reactive solvent, preferably a polar solvent which has a boiling point in the range of from 60°C to 150°C. Solvents which can be suitably employed include chlorinated hydrocarbons, such as ethylene chloride, methylene chloride or chloroform; chlorinated aromatic compounds such as 1,2,4-trichlorobenzene, or o-dichlorobenzene; ethereal solvents such as tetrahydrofuran or p-dioxane; aromatic solvents, such as benzene, toluene or xylene; or alcoholic solvents such as ethanol or butanol. Especially preferred solvents are those which are known to promote nucleophilic reactions, such as dimethylsulfoxide and dimethylformamide.

As indicated above, the alkyl esters (4) can cyclize spontaneously to form the compounds (1a) and (1b). This cyclization is enhanced by the presence of a catalytic quantity of a strong base, such as sodium or potassium tert-butoxide and in the presence of a polar solvent such as dimethylsulfoxide or dimethylformamide. The isomers (1a) and (1b) are obtained approximately in equimolecular amounts. Variations in reaction conditions can alter this ratio to favor one or the other isomer.

The alkyl esters of 3-[(N-alkyl)ureido]propionic acid (2) can be prepared by the reaction of an alkyl ester of 3-aminopropionic acid (5) with an alkyl isocyanate (6), containing a suitable leaving group as illustrated below. The symbols R, R₁, R₂, n and Y have the values previously described.

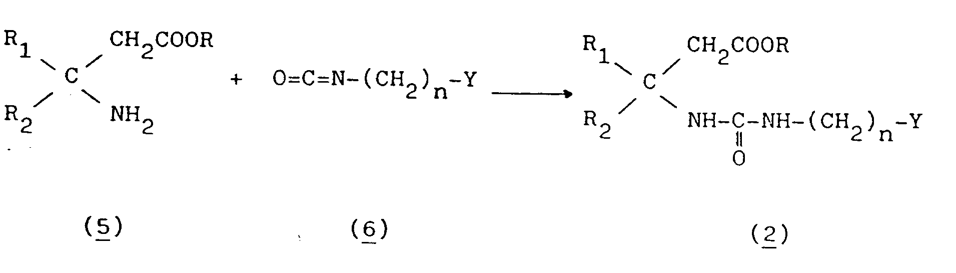

Approximately equimolar amounts of reactants (5) and (6) are employed. Inasmuch as both reactants are liquids at room temperature, the reaction can be conducted by simple mixing. Preferably, however, the use of a suitable aprotic solvent is employed. Suitable aprotic solvents include chlorinated hydrocarbons, such as methylene chloride, ethylene chloride or carbon tetrachloride; aromatic hydrocarbons such as benzene or toluene; ethereal solvents such as diethylether, tetrahydrofuran or p-dioxane and esters of organic acids such as ethyl acetate.

The reaction is exothermic and can be conducted at room temperature employing suitable means for cooling. Depending upon the reactants utilized, their concentration and the particular solvents employed, the reaction can be conducted at temperatures ranging from -30°C to ambient temperatures for periods of time ranging from 30 minutes to 3 hours. Preferably temperatures from -20°C to 0°C are employed, with reaction times ranging from 30 minutes to 1 hour. Removal of the solvents from the reaction mixture, generally via evaporation, leaves the desired alkyl esters of 3-[(N-alkyl)ureido]-propionic acid, which can then be utilized in the manner previously indicated.

Alternatively, the alkyl esters of 3-[N-[ω-[4-(2-pyrimidinyl)-1-piperazinyl]alkyl]ureido]propionic acid (4) can be prepared via the nucleophilic condensation of an alkyl ester of 3-isocyanopropionic acid of formula (7), with an ω-[4-(2-pyrimidinyl)-1-piperazinyl]alkylamine (8) as shown below. The symbols R, R₁, R₂ and n have the same values previously assigned. 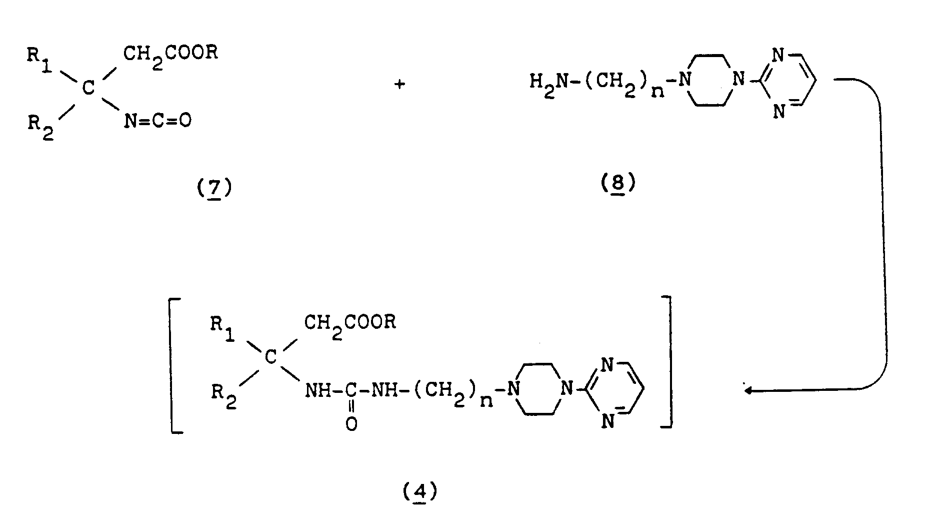
This nucleophilic condensation is preferably conducted using equimolar amounts of the alkyl esters of 3-isocyanopropionic acid (7) with the ω-[4-(2-pyrimidinyl)-1-piperazinyl]alkylamine (8) under essentially the same reaction conditions for the reaction previously described involving the converse functionalities, i.e., the reaction of an alkyl ester of 3-aminopropionic acid (5) with the alkyl isocyanate (6).

The compounds of formula (1) possess useful antianxiety properties. Anxiolytic properties are indicated using 5-HT_{1A} in vitro receptor binding studies, see Middlemiss et al., Eur. J. Pharmacol., 90, 151-3 (1983) and Glaser et al., Arch. Pharmacol., 329, 211-215 (1985). Additionally, the anxiolytic properties for the compounds described herein can be demonstrated in vivo, utilizing a rat licking test, which is a recognized animal model for anxiety utilized by those skilled in the art, see Vogel et al., Psychopharmacologia, 21, 1-7 (1971).

The compounds of this invention can be administered either orally, subcutaneously, intravenously, intramuscularly, intraperitoneally or rectally. The preferred route of administration is oral. The amount of compound to be administered can be any effective amount and will, of course, vary depending upon the patient, the mode of administration and the severity of the anxiety to be treated. Repetitive daily administration of the compounds may be desirable, and will vary depending upon the patient's condition and the particular mode of administration.

For oral administration, an anxiolytic effective amount of compound can range from 0.005 to 10 mg/kg of patient body weight per day, preferably from 0.05 to 5 mg/kg of patient body weight per day. Pharmaceutical compositions in unit dose form contain from 1 to 50 mg of active ingredient and can be administered one or more times per day.

The compounds can be formulated into solid or liquid preparations such as capsules, pills, tablets, lozenges, melts, powders, solutions, suspensions or emulsions. Solid dosage unit forms generally employed include capsules or tablets. Capsules can be of the ordinary gelatin type which contain additional excipients such as surfactants, lubricants and inert fillers such as lactose, sucrose and cornstarch. Tablets containing compounds of formula (1) can be formed with conventional tablet bases such as lactose, sucrose, and cornstarch in combination with binders, such as acacia, cornstarch or gelatin, disintegrating agents such as potato starch or alginic acid, and lubricants such as stearic acid or magnesium stearate.

For parenteral administration, an anxiolytic effective amount of compound ranges from about 0.005 to 10 mg/kg of patient body weight per day, preferably from about 0.05 to 5 mg/kg of patient body weight per day. A parenteral composition in unit dose form contains from 0.1 mg to 10 mg of active ingredient and can be administered one or more times daily.

The compounds may be administered as injectable dosages of a solution or a suspension of the compound in a physiologically acceptable diluent with or without a pharmaceutical carrier. Suitable diluents or carriers include sterile liquids such as water or oils, with or without the addition of surfactants or other pharmaceutically acceptable adjuvants. Illustrative of various oils that can be employed in the practice of this invention are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solution.

The following examples illustrate the preparation of representative compounds employed in the practise of this invention, but are not intended to limit the invention in any way thereto.

### EXAMPLE I

### 2-[4-[4-(2-Pyrimidinyl)-1-piperazinyl]butylimino]-tetranydro-4,4-dimethyl-6H-1,3-oxazine-6-one

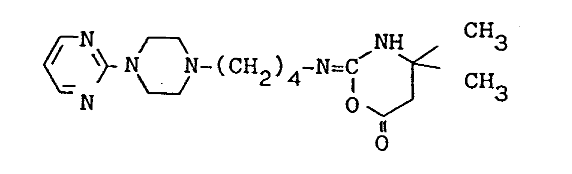

### 4-Bromobutylisocyanate

A solution of 5-bromopentanoic acid (36.2g) in thionyl-chloride (15.7ml) is stirred for 2 hours at room temperature and the excess thionyl chloride removed by evaporation. The acid chloride residue is dissolved in acetone (50ml) and the solution slowly added to a stirred suspension of sodium azide (13g) in water (15ml) at 0°C. After stirring for an additional 30 minutes, the reaction mixture is extracted with ether. The ether layer is washed several times with a saturated sodium bicarbonate solution, dried over MgSO₄, and evaporated to yield 5-bromopentanoyl azide (29g) as an oil. A solution of the acid azide in benzene is refluxed for 2 hours and then distilled to yield 4-bromobutylisocyanate, bp 110°C/15mm (15.1g).

### Ethyl 3-[(4-Bromobutyl)aminocarbonylamino]-3-methylbutanoate

A solution of 4-bromobutylisocyanate (15.1g) in methylene chloride (50ml) is added to a stirred solution of ethyl 3-amino-3-methylbutanoate (12.3g), dissolved in methylene chloride (50ml) at -20°C. The mixture is stirred for 30 minutes at -20°C and then for an additional 2 hours at room temperature. Evaporation of the solvent yields a residue of ethyl 3-[(4-bromobutyl)aminocarbonylamino]-3-methylbutanoate (26g).

### 2-[4-[4-(2-Pyrimidinyl)-1-piperazinyl]butylimino]tetrahydro-4,4-dimethyl-6H-1,3-oxazine-6-one

A mixture of ethyl 3-[(4-bromobutyl)aminocarbonylamino]-3-methylbutanoate (3.21g), 1-(2-pyrimidinyl)piperazine (1.64g), potassium carbonate (1.38g) and dimethylformamide (50ml) is heated at 100°C for 3 hours, left overnight at room temperature, filtered, and evaporated to dryness. The residue is chromatographed on silica gel using a mixture of ethyl acetate-ethanol (90:10) as the eluent to yield 2-[4-[4-(2-pyrimidinyl)-1-piperazinyl]butylimino]-tetranydro-4,4-dimethyl-6H-1,3-oxazine-6-one, m.p. 124-6°C when crystallized from aqueous methanol (Rf 0.3) and 1-[4-[4-(2-pyrimidinyl)-1-piperazinyl]butyl]tetrahydro-6,6-dimethylpyrimidine-2,4-dione, as an oil (Rf 0.2).

### EXAMPLE II

### 2-[4-[4-(2-Pyrimidinyl)-1-piperazinyl]butylimino]-tetrahydro-4,4-dimethyl-6H-1,3-oxazine-6-one

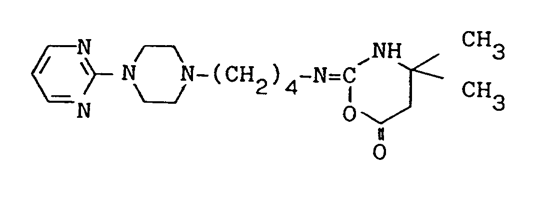

### 2-Ethoxycarbonyl-1,1-dimethylethylisocyanate

A solution of trichloromethyl chloroformate (4.1g) in methylene chloride (15ml) is slowly added to a stirred mixture of ethyl-3-amino-3-methylbutanoate (3g), triethylamine (6.6g) and methylene chloride (30ml) at 5°C. The mixture is stirred overnight at room temperature, refluxed for 3 hours, cooled and anhydrous ether added thereto. The precipitated triethylamine hydrochloride is filtered and the filtrate evaporated to yield 2-ethoxycarbonyl-1,1-dimethylethylisocyanate as an oil (3.5g).

### Ethyl 3-[4-[4-(2-pyrimidinyl)-1-piperazinyl]butyl]aminocarbonylamino-3-methylbutanoate

A solution of the above isocyanate (3.5g) in methylene chloride (10ml) is added to a stirred solution of 4-[4-(2-pyrimidinyl)-1-piperazinyl]butylamine (4.15g) in methylene chloride (10ml) at room temperature. A slight exothermic reaction is observed. After stirring for 30 minutes the methylene chloride is evaporated, the residue is dissolved in a one Normal solution of HCl (50ml), and the solution is extracted with ethyl acetate. The aqueous layer is made alkaline with saturated potassium carbonate and extracted with ether. Distillation of the dried ether extract yields ethyl 3-[4-[4-(2-pyrimidinyl)-1-piperazinyl]butyl]aminocarbonylamino-3-methylbutanoate as a syrup (5.4g).

### 2-[4-[4(2-pyrimidinyl)-1-piperazinyl]butylimino]-tetrahydro-4,4-dimethyl-6H-1,3-oxazine-6-one.

A solution of potassium tert butoxide (1g) in ethanol (5ml) is added to a solution of ethyl 3-[4-[4-(2-pyrimidinyl)-1-piperazinyl]butyl]aminocarbonylamino-3-methylbutanoate (5.4g) in ethanol (10ml) and the stirred mixture refluxed for 30 minutes. The ethanol is evaporated, the residue is treated with water (50ml) and the oil that forms is extracted with methylene chloride. Evaporation of the combined, dried methylene chloride extracts yields a syrup which partially crystallises. The syrup is treated with ether and the crystals are removed by filtration (2g). A solution of the crystals in aqueous methanol is treated with charcoal, filtered and is allowed to recrystallize to yield 2-[4-[4-(2-pyrimidinyl)-1-piperazinyl]butylimino]-tetrahydro-4,4-dimethyl-6H-1,3-oxazine-6-one, m.p. 124-6°C (1g).

### EXAMPLE III

### In vitro Determination of Anxiolytic Properties via 5-HT_{1A}Binding

Radioligand binding studies of the 5-HT_{1A} recognition sites are conducted in the following manner. Male normotensive Sprague-Dawley rat frontal cortex is dissected, frozen in liquid nitrogen and stored at -20°C until needed. Tissues from 4-8 rats are pooled and homogenized in 70 vol Tris-HCl buffer (50mM, pH 7.7), using a kinematical Polytron (setting 2/3 max speed, 20 sec). The homogenate is centrifuged (36500 x g for 10 min), the pellet re-homogenised in the same volume of buffer and the process is repeated two more times. Between the second and third centrifugations the tissue homogenate is incubated at 37°C for 10 min. The final pellet is suspended in the same volume of Tris buffer containing 10 M pargyline, 5.7mM CaCl₂ and 0.1% ascorbic acid. This suspension is incubated for 10 min at 37°C and then stored on ice until used in the binding assay.

Tissue homogenate (0.7ml), radioactive ligand (0.1ml) and the appropriate concentration of test compound (0.1ml), together with buffer to a final volume of 1ml are incubated at 37°C for 15 min. Incubations are terminated by rapid filtration through Whatman GF/B filters followed by three 5ml washes with ice-cold Tris-HCl buffer (50mM, pH 7.0). Radioactivity is measured following extraction into Aquasol-Z (NEN) at an efficiency of 45-50%. The radioligand used to label the 5-HT_{1A} recognition sites and its concentration is[³H]-8-hydroxy-2-(di-n-propylamino)-tetralin, [³H]-8-OH-DPAT, 1mM.

Following essentially the above procedure, the compound 2-[4-[4-(2-pyrimidinyl)-1-piperazinyl]butylimino]-tetrahydro-4,4-dimethyl-6H-1,3-oxazine-6-one, (Compound B) is tested. The results are expressed as pIC₅₀ (log₁₀ concentration of test compound which inhibits specific binding by 50%).

| Test Compound | 5-HT_{1A} Binding Affinity Rat Brain cortex |
|---|---|
| Compound B | 6.32 |
| Buspirone | 7.52 |

## Claims (Claims for the following Contracting State(s): BE, CH, DE, FR, GB, IT, LI, LU, NL, SE)

1. A 4-(2-pyrimidinyl)-1-piperazinyl heterocyclic carbonyl derivative having the formula (1): 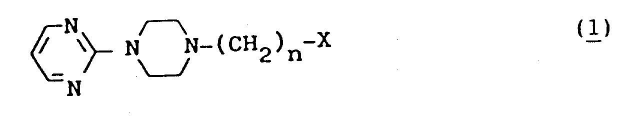 wherein
n is an integer from 2 to 5;
X is the radical 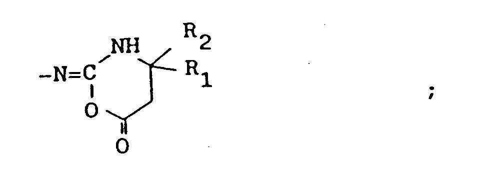
R₁ and R₂ are each methyl or when taken together form a tetramethylene or pentamethylene ring;
and the pharmaceuticallly acceptable acid addition salts thereof.

2. A compound according to Claim 1, which is 2-[4-[4-(2-pyrimidinyl)-1-piperazinyl]butylimino]-tetrahydro-4,4-dimethyl-6H-1,3-oxazine-6-one and the pharmaceutically acceptable salts thereof.

3. A process for the preparation of a 4-(2-pyrimidinyl)-1-piperazinyl heterocyclic carbonyl derivative according to Claim 1 which comprises reacting an alkyl ester of 3-[(N-alkyl)ureido]propionic acid having the formula 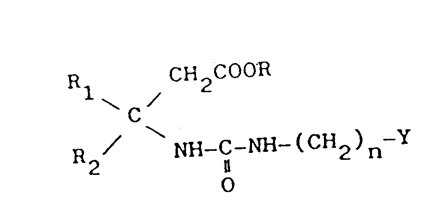 wherein n, R₁ and R₂ are as described in Claim 1, R is a lower alkyl group having 1 to 4 carbon atoms and Y is an appropriate leaving group, with 1-(2-pyrimidinyl)-piperazine to form an alkyl ester of 3-[[N-[ω-[4-(2-pyrimidinyl)]-1-piperazinyl]alkyl]ureido]propionic acid; cyclizing said alkyl ester of (2-pyrimidinyl)-l-piperazinylalkylureidopropionic acid, and isolating the desired 4-(2-pyrimidinyl)-1-piperazinyl heterocyclic carbonyl derivative therefrom.

4. A compound of Claim 1 or 2 for use as a medicine.

5. Use of a compound of Claim 1 or 2 for preparing a medicament for anxiolytic use.

6. A pharmaceutical composition which comprises a compound of Claim 1 or 2 in admixture with a pharmaceutically acceptable carrier.

## Claims (Claims for the following Contracting State(s): AT, ES, GR)

1. A process for the preparation of a 4-(2-pyrimidinyl)-1-piperazinyl heterocyclic carbonyl derivative having the formula (1): 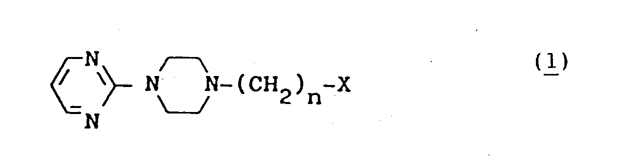 wherein
n is an integer from 2 to 5;
X is the radical 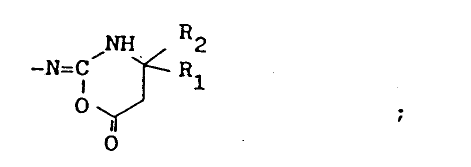
R₁ and R₂ are each methyl or when taken together form a tetramethylene or pentamethylene ring;
and the pharmaceuticallly acceptable acid addition salts thereof, which comprises reacting an alkyl ester of 3-[N-alkyl)ureido]propionic acid having the formula 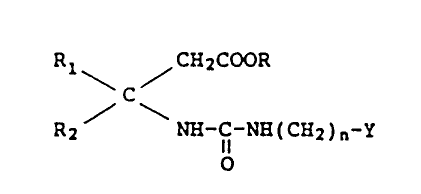 wherein n, R₁ and R₂ are as described above, R is a lower alkyl group having 1 to 4 carbon atoms and Y is an appropriate leaving group, with 1-(2-pyrimidinyl)-piperazine to form an alkyl ester of 3-[[N-[ω-[-(2-pyrimidinyl)]-1-piperazinyl]alkyl]ureido]propionic acid; cyclizing said alkyl ester of (2-pyrimidinyl)-1-piperazinylalkylureidopropionic acid, and isolating the desired 4-(2-pyrimidinyl)-1-piperazinyl heterocyclic carbonyl derivative therefrom.

2. A process for preparing a compound of Claim 1 which comprises allowing to cyclize an alkyl ester of (2-pyrimidinyl)-1-piperazinylalkylureidopropionic acid of formula (4): 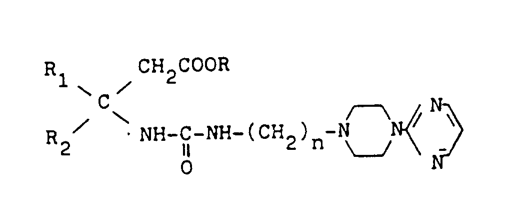 wherein R₁ and R₂ are defined as in Claim 1 and R represents a lower alkyl group having 1 to 4 carbon atoms in a polar solvent optionally in the presence of catalytic amounts of a strong base.

3. A process according to Claim 1 or 2 for preparing a compound of formula (1) which is 2-[4-[4-(2-pyrimidinyl)-1-piperazinyl]butylimino]-tetrahydro-4,4-dimethyl-6H-1,3-oxazine-6-one and the pharmaceutically acceptable salts thereof.

4. Use of a compound of Claim 1,2 or 3 for preparing a medicament for anxiolytic use.

## Patentansprüche (Patentansprüche für folgende(n) Vertragsstaat(en): BE, CH, DE, FR, GB, IT, LI, LU, NL, SE)

1. Heterocyclisches 4-(2-Pyrimidinyl)-1-piperazinyl-Carbonylderivat der Formel (1): 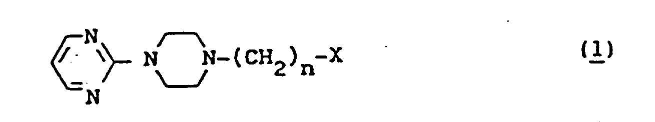 in der
n eine ganze Zahl von 2 bis 5 bedeutet;
X den Rest der Formel 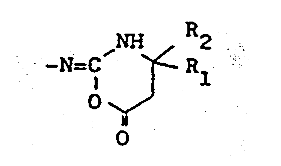
bedeutet, in der R₁ und R₂ jeweils Methylgruppen darstellen oder zusammengenommen einen Tetramethylen- oder Pentamethylenring bilden; und die pharmazeutisch verträglichen Säureadditionssalze davon.

2. Verbindung nach Anspruch 1, nämlich 2-[4-[4-(2-Pyrimidinyl)-1-piperazinyl]butylimino]-tetrahydro-4,4-dimethyl-6H-1,3-oxazin-6-on und die pharmazeutisch verträglichen Salze davon.

3. Verfahren zur Herstellung eines heterocyclischen 4-(2-Pyrimidinyl)-1-piperazinyl-Carbonylderivats nach Anspruch 1, umfassend die Umsetzung eines Alkylesters von 3-[(N-Alkyl)ureido]propionsäure der Formel 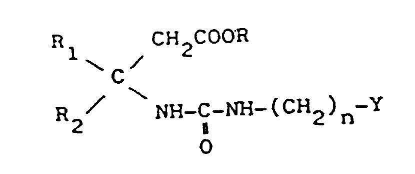 in der n, R₁ und R₂ die in Anspruch 1 beschriebene Bedeutung besitzen, R einen Niederalkylrest mit 1 bis 4 Kohlenstoffatomen darstellt und Y eine geeignete Austrittsgruppe bedeutet, mit 1-(2-Pyrimidinyl)piperazin unter Herstellung eines Alkylesters von 3-[[N-[ω-[4-(2-Pyrimidinyl)]-1-piperazinyl]alkyl]ureido]propionsäure;
Cyclisieren des Alkylesters von (2-Pyrimidinyl)-1-piperazinylalkylureidopropionsäure und Isolieren des gewünschten heterocyclischen 4-(2-Pyrimidinyl)-1-piperazinyl Carbonylderivats davon.

4. Verbindung nach Anspruch 1 oder 2 zur Verwendung als Arzneimittel.

5. Verwendung einer Verbindung nach Anspruch 1 oder 2 zur Herstellung eines Arzneimittels, das als Anxiolytikum Verwendung findet.

6. Arzneimittel, umfassend eine Verbindung nach Anspruch 1 oder 2 im Gemisch mit einem pharmazeutisch verträglichen Träger.

## Patentansprüche (Patentansprüche für folgende(n) Vertragsstaat(en): AT, ES, GR)

1. Verfahren zur Herstellung eines heterocyclischen 4-(2-Pyrimidinyl)-1-piperazinyl-Carbonylderivats der Formel (1) 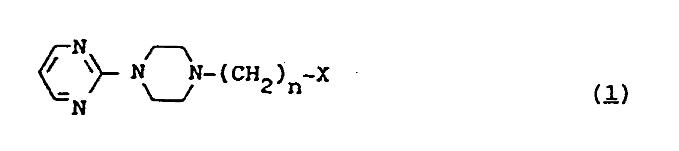 in der
n eine ganze Zahl von 2 bis 5 bedeutet;
X den Rest der Formel 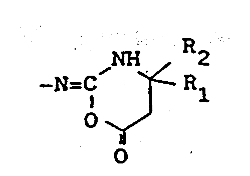
bedeutet, in der R₁ und R₂ jeweils Methylgruppen darstellen oder zusammengenommen einen Tetramethylen- oder Pentamethylenring bilden;
und den pharmazeutisch verträglichen Säureadditionssalzen davon, umfassend die Umsetzung eines Alkylesters von 3-[(N-Alkyl)ureido)propionsäure der Formel 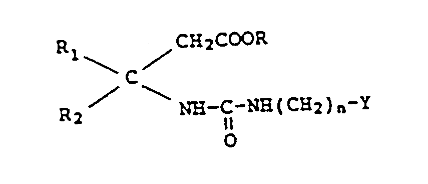 in der n, R₁ und R₂ die in Anspruch 1 beschriebene Bedeutung besitzen, R einen Niederalkylrest mit 1 bis 4 Kohlenstoffatomen darstellt und Y eine geeignete Austrittsgruppe bedeutet, mit 1-(2-Pyrimidinyl)piperazin unter Herstellung eines Alkylesters von 3-[[N-[ω-[4-(2-Pyrimidinyl)]-1-piperazinyl]alkyl]ureido]propionsäure;
Cyclisieren des Alkylesters von (2-Pyrimidinyl)-1-piperazinylalxylureidopropionsäure und Isolieren des gewünschten heterocyclischen 4-(2-Pyrimidinyl)-1-piperazinyl-Carbonylderivats davon.

2. Verfahren zur Herstellung einer Verbindung nach Anspruch 1, umfassend das Cyclisieren eines Alkylesters von (2-Pyrimidinyl)-1-piperazinylalkylureidopropionsäure der Formel (4) 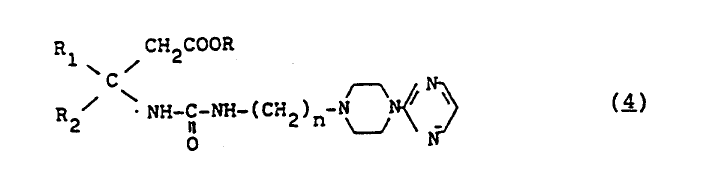 in der R₁ und R₂ wie in Anspruch 1 definiert sind und R einen Niederalkylrest mit 1 bis 4 Kohlenstoffatomen bedeutet, in einem polaren Lösungsmittel, gegebenenfalls in Gegenwart katalytischer Mengen einer starken Base.

3. Verfahren nach Anspruch 1 oder 2 zur Herstellung einer Verbindung nach Formel (1) nämlich 2-[4-[4-(2-Pyrimidinyl)-1-piperazinyl]butylimino]-tetrahydro-4,4-dimethyl-6H-1,3-oxazin-6-on und den pharmazeutisch verträglichen Salzen davon.

4. Verwendung einer Verbindung nach Anspruch 1, 2 oder 3 zur Herstellung eines Arzneimittels, das als Anxiolytikum Verwendung findet.

## Revendications (Revendications pour l'(les) Etat(s) contractant(s) suivant(s): BE, CH, DE, FR, GB, IT, LI, LU, NL, SE)

1. Dérivé hétérocyclique carbonylé substitué par un groupe 4-(2-pyrimidinyl)-1-pipérazinyle répondant à la formule (1) : 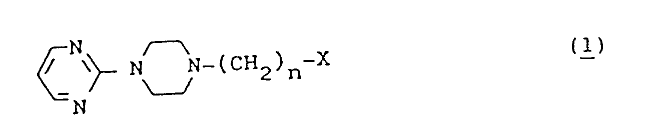 dans laquelle
n est un entier de 2 à 5 ;
X est le radical 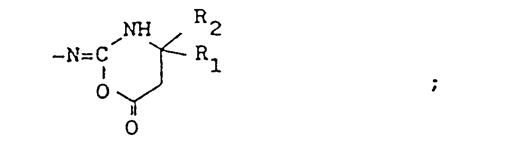
R₁ et R₂ sont chacun un méthyle ou, ensemble, forment un cycle tétraméthylène ou pentaméthylène ; et ses sels d'addition d'acides pharmaceutiquement acceptables.

2. Composé selon la revendication 1 qui est la 2-[4-[4-(2-pyrimidinyl)-1-pipérazinyl]butylimino]tétrahydro-4,4-diméthyl-6H-1,3-oxazine-6-one et ses sels pharmaceutiquement acceptables.

3. Procédé pour la préparation d'un dérivé hétérocyclique carbonylé substitué par un groupe 4-(2-pyrimidinyl)-1-pipérazinyle selon la revendication 1, qui comprend la réaction d'un ester d'alkyle d'acide 3-[(N-alkyl)uréido]propionique de formule 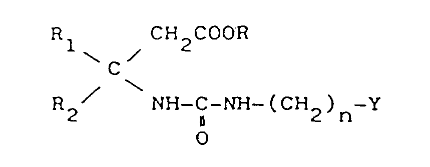 dans laquelle n, R₁ et R₂ sont comme décrits dans la revendication 1, R est un groupe alkyle inférieur ayant 1 à 4 atomes de oarbone et Y est un groupe labile approprié, avec la 1-(2-pyrimidinyl)pipérazine, pour former un ester d'alkyle d'acide 3-[[N-[ω-[4-(2-pyrimidinyl)]-1-pipérazinyl]alkyl)uréido]propionique ; la cyclisation dudit ester d'alkyle d'acide 2-(pyrimidinyl)-1-pipérazinylalkyluréidopropionique et l'isolement du dérivé hétérocyclique carbonylé substitué par un groupe 4-(2-pyrimidinyl)-1-pipérazinyle.

4. Composé selon la revendication 1 ou 2 pour l'utilisation comme médicament.

5. Utilisation d'un composé selon la revendication 1 ou 2 pour la préparation d'un médicament à usage anxiolytique.

6. Composition pharmaceutique qui comprend un composé selon la revendication 1 ou 2 en mélange avec un véhicule pharmaceutiquement acceptable.

## Revendications (Revendications pour l'(les) Etat(s) contractant(s) suivant(s): AT, ES, GR)

1. Procédé pour la préparation d'un dérivé hétérocyclique carbonylé substitué par un groupe 4-(2-pyrimidinyl)-1-pipérazinyle répondant à la formule (1) : 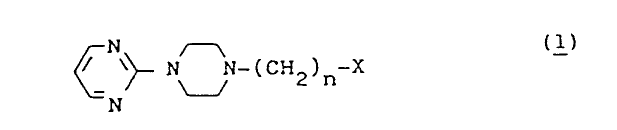 dans laquelle
n est un entier de 2 à 5 ;
X est le radical 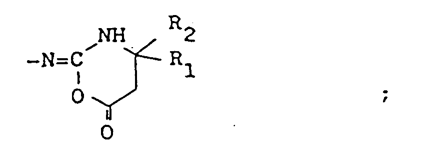
R₁ et R₂ sont chacun un méthyle ou, ensemble, forment un cycle tétraméthylène ou pentaméthylène ;
et ses sels d'addition d'acides pharmaceutiquement acceptables, qui comprend la réaction d'un ester d'alkyle d'acide 3-[(N-alkyl)uréido] propionique de formule 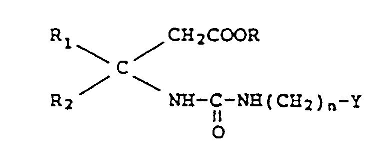 dans laquelle n, R₁ et R₂ sont comme décrits ci-dessus, R est un groupe alkyle inférieur ayant 1 à 4 atomes de carbone et Y est un groupe labile approprié, avec la 1-(2-pyrimidinyl)pipérazine, pour former un ester d'alkyle d'acide 3-[[N-[ω-[4-(2-pyrimidinyl)]-1-pipérazinyl]alkyl]uréido]propionique ; la cyclisation dudit ester d'alkyle d'acide 2-(pyrimidinyl)-1-pipérazinylalkyluréidopropionique et l'isolement du dérivé hétérocyclique carbonylé substitué par un groupe 4-(2-pyrimidinyl)-1-pipérazinyle.

2. Procédé pour préparer un composé selon la revendication 1, qui consiste à cycliser un ester d'alkyle d'acide (2-pyrimidinyl)-1-pipérazinylalkyluréidopropionique de formule (4) : 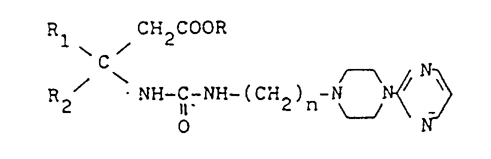 dans laquelle R₁ et R₂ sont comme définis dans la revendication 1 et R représente un groupe alkyle inférieur ayant 1 à 4 atomes de carbone, dans un solvant polaire, éventuellement en présence de quantités catalytiques d'une base forte.

3. Procédé selon la revendication 1 ou 2 pour la préparation d'un composé de formule (1) qui est la 2-[4-[4-(2-pyrimidinyl)-1-pipérazinyl]butylimino]tétrahydro-4,4-diméthyl-6H-1,3-oxazine-6-one et ses sels pharmaceutiquement acceptables.

4. Utilisation d'un composé selon la revendication 1, 2 ou 3 pour la préparation d'un médicament à usage anxiolytique.
